# EUROPEAN PATENT SPECIFICATION

(11) **EP 0 580 847 B1**
(45) Date of publication and mention of the grant of the patent: **31.03.1999**
(21) Application number: 93905038.1
(22) Date of filing: 12.02.1993
(51) Int. Cl.: B01J 29/00, B01J 29/06, B01J 29/12

(54) **CATALYST AND PROCESS FOR HYDROCARBON DEHYDROGENATION**
KATALYSATOR UND VERFAHREN ZUR DEHYDRIERUNG VON KOHLENWASSERSTOFFEN
CATALYSEUR ET PROCEDE POUR LA DESHYDROGENATION D'HYDROCARBURES

(30) Priority: 13.02.1992 US 835231; 14.09.1992 US 944499
(43) Date of publication of application: 02.02.1994
(60) Divisional application: 98116920.4
(73) Proprietor: AMOCO CORPORATION, Chicago, IL 60680-0703 (US)
(72) Inventor: KAMINSKY, Mark, P., Winfield, IL 60190 (US); FROMENT, Gilbert, Fernand, Alphonse, Deurle B-9831 St. Martens Latem (BE); DEHERTOG, Wilfried, Jozef, Hippolyte, B-3080 Tervuren (BE)
(74) Representative: Ritter, Stephen David
(86) International application number: US9301273
(87) International publication number: WO9315835

(56) References cited:
- EP-A- 0 186 479
- US-A- 4 990 710
- US-A- 5 057 635
- US-A- 5 073 673

## Description

### Background of the Invention

This invention relates to a process for the dehydrogenation of one or more C₃ to C₆ saturated hydrocarbons over a catalyst which comprises a platinum-loaded, crystalline aluminosilicate molecular sieve exhibiting the MFI crystal structure which is preferably in the sodium form, and more particularly, to a process for the dehydrogenation of one or more of propane, butane, pentane, or hexane over a supported or unsupported platinum-loaded, crystalline aluminosilicate molecular sieve exhibiting the MFI crystal structure which is preferably in the sodium form and preferably has a silicon-to-aluminum atomic ratio of at least 800.

In the past various molecular sieve compositions natural and synthetic have been found to be useful for a number of hydrocarbon conversion reactions. Among these are alkylation, aromatization, dehydrogenation and isomerization. Among the sieves which have been used are Type A, X, Y and those of the MFI crystal structure, as shown in Atlas of Zeolite Structure Types, Second Revised Edition 1987, published on behalf of the Structure Commission of the International Zeolite Associates and incorporated by reference herein. Representative of the last group are ZSM-5 and AMS borosilicate molecular sieves.

The catalytic dehydrogenation of alkanes such as propane, butane, pentane, and hexane are particularly important industrial processes and optimizing the catalyst lifetime, conversion and selectivity to olefins has been the subject of a great deal of effort. For example, in European Patent Application 0186479 to Mobil, a ZSM-5 type of sieve with a high (7000) silica/alumina ratio and containing a substantial platinum content was made by loading the sodium form of the sieve with about 0.5% by weight platinum, reducing the platinum with a mixture of nitrogen and 1-hexene, and finally exchanging the reduced sieve with potassium hydroxide. Example 5 shows the use of such a platinum-loaded, base exchanged sieve for the dehydrogenation of propane. Conversion and selectivity to propane at 550-575°C are said to be 30 and 85% respectively.

Heterogeneous catalysts comprising platinum group metals for the dehydrogenation of liquid or gaseous hydrocarbons have been previously described. Representative of the prior art relating to platinum group metal catalysts are U.S. Pat. Nos. 3,531,543; 3.745,112; 3,892,657; 3,909,451; 4,101,593; 4,210,769; 4,329,258; 4,363,721; 4,438,288; 4,665,267; and British Patent No. 1,499,297. Generally, in addition to the platinum group metal, there is employed a porous support and an additional component specifically selected for the purpose of improving the activity and/or selectivity and/or stability of the catalyst. The additional component is typically an alkali metal or an alkaline earth metal. A large number of porous supports are reported including crystalline zeolite silicates.

Further examples of prior art catalysts include those disclosed in EP 0 186 479 wherein a so-called "shape selective zeolite catalyst composition" is produced by calcining a crystalline zeolite having an Si:Al ratio of at least 12 and a constraint index of 1 to 12 at a temperature of 200°C to 600°C for 1-48 hours, contacting the calcine zeolite with an aqueous solution of a Group VIII metal compound, thermally treating the resulting Group VIII metal containing zeolite at 150°C to 550°C and base exchanging the zeolite with Group 1A metal cations. The catalysts are stated to possess "an unusual capability to ion exchange Group VIII metal" and that "several fold excess of Group VIII metal cations can be incorporated in the zeolite, over the quantity theoretically projected on the basis of the aluminum content of the zeolite".

US 4,990,710 describes Group VIII metal modified non-acidic tin containing microporous crystalline silicate catalysts and their use in the catalytic dehydrogenation of paraffins. Hydrogenolysis to methane is stated to constitute a major competing side reaction.

Now a platinum-containing, aluminosilicate molecular sieve catalyst made by a simplified procedure has been found which is able to dehydrogenate, for example, propane at a conversion equal to the thermodynamic equilibrium value at a particular temperature with a selectivity to the corresponding olefin close to one hundred percent while exhibiting minimal coking leading to a long catalyst run length.

### Summary of the Invention

A platinum-containing catalyst composition suitable for dehydrogenating C₃ to C₆ alkanes to alkenes, which composition contains 0.002 wt% to less than 0.01 wt% platinum, and is prepared by the process comprising contacting a crystalline aluminosilicate sieve exhibiting the MFI crystal structure and having a platinum exchange capacity of no more than about 200 parts per million by weight platinum, with at least one platinum compound to form a platinum-treated sieve; and thereafter heat-treating the platinum-treated sieve to form the platinum-containing catalyst composition.

In another aspect, this invention is a dehydrogenation process comprising contacting under hydrocarbon conversion conditions at least one C₃ to C₆ alkane with a platinum-containing catalyst composition, wherein the platinum-containing composition is prepared by the process comprising contacting a crystalline aluminosilicate sieve exhibiting the MFI crystal structure and having a platinum exchange capacity of no more than about 1000 parts per million by weight platinum, with at least one platinum compound to form a platinum-treated sieve; and thereafter heat-treating the platinum-treated sieve to form the platinum-containing catalyst composition.

### Brief Description of the Drawing

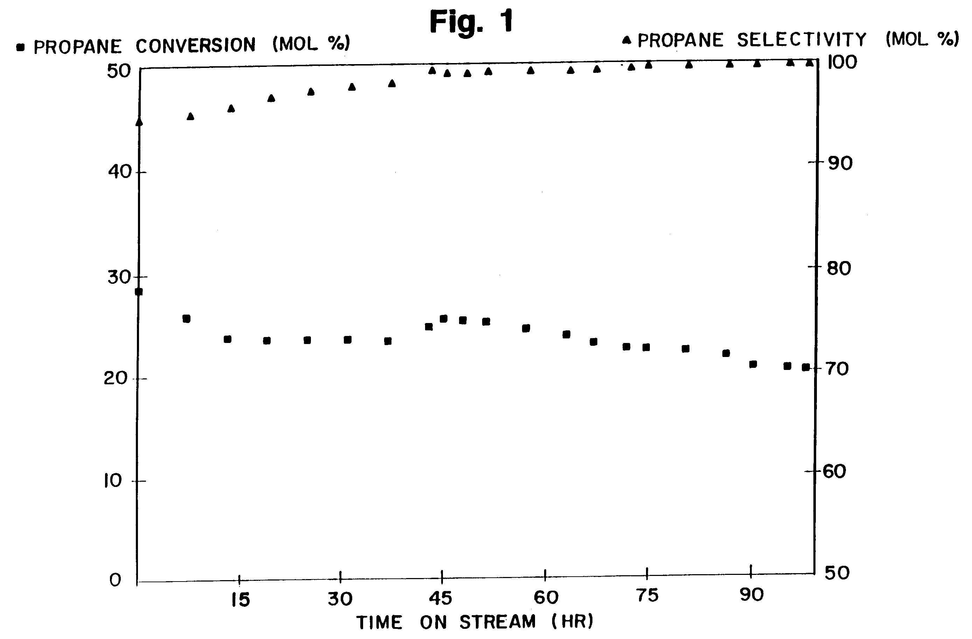

Figure 1 shows the conversion and selectivity as a function of time on stream for catalyzed propane dehydrogenation to propylene at a weight hourly space velocity (WHSV) of 1.5 hr⁻ ¹, 1.05 bar (105 kPa) and 540°C. The catalyst is a platinum-loaded, essentially sodium form crystalline aluminosilicate molecular sieve having the MFI crystal structure and a Si/Al ratio greater than about 800.

### Detailed Description of the Invention

The hydrocarbon feedstock useful in the dehydrogenation process described herein is at least one C₃ to C₆ alkane. More preferably, it is propane, a butane, a pentane, a hexane, or a mixture of two or more of such aliphatic hydrocarbons.

The highly siliceous crystalline molecular sieves useful in this invention can be prepared by crystallizing an aqueous mixture, at a controlled pH, of sources for cations, an oxide of aluminum, an oxide of silicon, and an organic template compound.

Typically, the mole ratios of the various reactants can be varied to produce the crystalline silicates of this invention. Specifically, the mole ratios of the initial reactant concentrations are indicated below:

| Reactants | Broad | Preferred | Most Preferred |
|---|---|---|---|
| SiO₂/Al₂O₃ | 500-5000 | 1200-4500 | 1600-4000 |
| R₂O+/[R₂O⁺+ M_{2/n}O] | 0.1-1.0 | 0.1-0.90 | 0.1-0.70 |
| OH⁻/SiO₂ | 0.01-11 | 0.01-2 | 0.01-1 |
| H₂O/OH⁻ | 10-4000 | 10-500 | 10-500 |

wherein R is an organic compound and M is at least one cation having the oxidation state n, such as an alkali or an alkaline earth metal cation or hydrogen. By regulation of the quantity of aluminum (represented as Al₂O₃) in the reaction mixture, it is possible to vary the SiO₂/Al₂O₃ molar ratio in the final product.

More specifically, the material useful in the present invention can be prepared by mixing a base, an aluminum oxide source,if required, and an organic template compound in water (preferably distilled or deionized). The order of addition usually is not critical although a typical procedure is to dissolve base and sodium aluminate, if required, in water and then add the template compound. Generally, the silicon oxide compound is added with intensive mixing such as that produced in a Waring blender and the resulting slurry is transferred to a closed crystallization vessel for a suitable time. After crystallization, the resulting crystalline product can be filtered, washed with water, dried, and calcined.

During preparation, acidic conditions should be avoided. When alkali metal hydroxides are used, the values of the ratio of OH⁻/SiO₂ shown above should furnish a pH of the system that broadly falls within the range of about 9 to about 13.5. Advantageously, the pH of the reaction system falls within the range of about 10.5±0.5.

Examples of materials affording silicon oxide useful in this invention include silicic acid, sodium silicate, tetraalkyl silicates and Aerosil-380, manufactured by Degussa AG. Typically, the oxide of aluminum source is sodium aluminate, although many silicas already contain sufficient aluminum impurity contents to result in materials useful in this invention.

Cations useful in formation of crystalline molecular sieves of this invention include alkali metal and alkaline earth metal cations such as sodium, potassium, lithium, calcium and magnesium. Since basic conditions are required for crystallization of the molecular sieve of this invention, the source of such cation usually is a hydroxide such as sodium hydroxide.

A large number of organic templates are useful in preparing the crystalline aluminosilicates and include alkylammonium cations or precursors thereof such as tetraalkylammonium compounds, especially tetra-n-propylammonium compounds. A useful organic template is tetra-n-propylammonium bromide. Diamines, such as hexamethylenediamine, can be used.

In a more preferred description of a typical preparation of this invention, suitable quantities of sodium hydroxide and silica are dissolved with intense mixing in distilled or deionized water followed by addition of the organic template. The sodium aluminate is then slowly added with mixing. The pH is adjusted to about 10.5±0.05 using a compatible acid such as sulfuric acid or base.

Alternatively, and more preferably, crystalline aluminosilicate molecular sieve can be prepared by crystallizing a mixture of sources for an oxide of silicon, an oxide of aluminum, an alkyl ammonium compound and sodium hydroxide such that the initial reactant molar ratios of water to silica range from about 1 to about 20, preferably about 3 to about 10 and most preferably from about 4 to about 6. In addition, preferable molar ratios for initial reactant silica to oxide of aluminum range from about 500 to about 5000, more preferably from about 1200 to about 4500 and most preferably from about 1600 to about 4000. The molar ratio of sodium hydroxide to silicon oxide should be above about 0.01, typically below 11, preferably between about 0.01 and about 2.0 and most preferably between about 0.01 and 1.0. The molar ratio of alkylammonium compound, such as tetra-n-propylammonium bromide, to silicon oxide can range from 0 to about 1 or above, typically above about 0.005, preferably about 0.01 to about 0.1, more preferably about 0.01 to about 0.1 and most preferably about 0.04 to about 0.08.

The resulting slurry is transferred to a closed crystallization vessel and reacted usually at a pressure at least the vapor pressure of water for a time sufficient to permit crystallization which usually is about 0.25 to about 20 days, typically about one to about ten days and preferably about one to about seven days, at a temperature ranging from about 100°C to about 250°C, preferably about 125°C to about 200°C. The crystallizing material can be stirred or agitated as in a rocker bomb. Since contamination of the sieve product by metal ions is to be avoided, the crystallization vessel is preferably lined with a material, such as a Teflon®, which may reduce or prevent unwanted metal ions from contaminating the sieve product. Other materials useful for lining the crystallization vessel include quartz, Torlon®, Nylon®, high density polyethylene, polypropylene, and the like. It is also desirable to use high purity sources of the silica and alumina (e.g., sodium aluminate) so that unwanted metal ions do not contaminate the sieve. Preferably, the crystallization temperature is maintained below the decomposition temperature of the organic template compound. Especially preferred conditions are carrying out the crystallization at about 150°C for about two to five days. Samples of material can be removed during crystallization to check the degree of crystallization and determine the optimum crystallization time.

The crystalline material formed can be separated and recovered by well-known means such as filtration with aqueous washing. This material can be mildly dried for anywhere from a few hours to a few days at varying temperatures, typically about 50-225°C, to form a dry cake which can then be crushed to a powder or to small particles and extruded, pelletized, or made into forms suitable for its intended use. Typically, materials prepared after mild drying contain the organic template compound and water of hydration within the solid mass and a subsequent activation or calcination procedure is necessary, if it is desired to remove this material from the final product. Typically, mildly dried product is calcined at temperatures ranging from about 260°C to about 850°C and preferably from about 475°C to about 700°C. Extreme calcination temperatures or prolonged crystallization times may prove detrimental to the crystal structure or may totally destroy it. Typically, the molecular sieve material is dried in a forced draft oven at about 110°C for about 16 hr and is then calcined in air in a manner such that the temperature rise does not exceed 125°C per hour until a temperature of about 550°C is reached. Calcination at this temperature usually is continued for about 4 to 16 hr. The resulting sieve may be further calcined at about 600°C to about 750°C, and typically at about 650°C. This higher temperature calcination is suitably conducted for about 1 to about 24 hrs.

The platinum-loaded molecular sieve of the invention preferably has a silicon/aluminum (Si/Al) atomic ratio greater than 800, more preferably between 800 and 2000, and most preferably between 800 and 1500.

The catalytically active platinum is placed onto the aluminosilicate sieve before incorporation into an inorganic matrix, if used, by an ion exchange technique. Before placing the catalytically active platinum compound onto the molecular sieve, it is most preferable that the sieve be essentially in the sodium form.

If the sodium form of the molecular sieve is not made directly in the sieve preparation, the original cation in the aluminosilicate sieve can be replaced by repeated ion exchange with sodium ion. It is preferred to make the sodium form of the sieve during preparation of the sieve. Ion exchange techniques are well-known in the art.

The catalyst composition of this invention is prepared by adding a platinum component to the crystalline molecular sieve.

The platinum may be added to the molecular sieve by any known manner that produces a relatively uniform distribution of the platinum component on the sieve. The preferred method, however, is by ion exchange. Such ion exchange involves contacting the molecular sieve one or more times with a solution of a platinum compound, typically at a temperature of about 20°C to about 100°C, and suitably for about 0.1 hr to about 24 hrs. For example, an aqueous solution of one or more of chloroplatinic acid, ammonium chloroplatinate, bromoplatinic acid, platinum trichloride, platinum tetrachloride hydrate, dinitrodiamino platinum, sodium tetranitroplatinate (II), and the like, can be used to add platinum to the sieve. Tetraaminoplatinum (II) chloride is preferred. After contacting the sieve with the solution of the platinum compound, the sieve is washed with fresh solvent, typically water, to remove excess platinum. The calcined molecular sieve has a platinum exchange capacity of no more than 500 ppm by weight. Most preferably, the calcined sieve should have a platinum exchange capacity of no more than about 200 ppm by weight.

As used herein, platinum exchange capacity is the weight amount of platinum that the molecular sieve can ion exchange with, i.e., the platinum that cannot be washed free from the sieve. The platinum exchange capacity is determined by contacting the molecular sieve with a solution of soluble platinum compound, wherein the solution contains a molar excess of platinum relative to the available exchange sites on the sieve. After a suitable time period of contacting the sieve with the platinum solution, the sieve is separated from the solution, and the excess solution is removed from the sieve, typically by washing the sieve with fresh solvent. The amount of platinum remaining on the sieve represents the exchange capacity of the sieve, and can be measured in parts per million by weight platinum, i.e., ppm of platinum relative to the initial sieve in its dry, calcined state. The solution of platinum compound used to add the platinum to the sieve is typically an aqueous solution of one or more soluble platinum compounds, such as those disclosed hereinabove. Tetraamineplatinum (II) chloride, however, is a preferred source of platinum. More specifically, the platinum exchange capacity can be determined by contacting 20 grams of the molecular sieve that has been previously calcined at 650°C for 8 hrs, with a solution of 0.1712 grams of tetraamineplatinum (II) chloride hydrate (0.005 gram platinum per gram of sieve) in 10 liters of doubly-distilled water for 24 hrs at room temperature. The resulting sieve is filtered from the platinum-containing solution, and washed with 1 liter of fresh doubly-distilled water per gram of sieve,and dried. The amount of platinum in the resulting sieve, as measured by ICP analysis or other analysis, is the exchange capacity of the sieve, and can be expressed as parts per million by weight platinum on the dry sieve used for the exchange.

The use of a sieve having the platinum exchange capacity described hereinabove provides for a catalyst that has a relatively low loading of platinum. Such a low loading of platinum provides for an economical catalyst because lower amounts of expensive platinum are used. However, the catalyst of this invention having a low loading of platinum is surprisingly effective as a catalyst for the dehydrogenation of aliphatic hydrocarbons. Without intending to be bound by a theory of operation, it is believed that the dehydrogenation catalysts disclosed herein having a relatively low loading of platinum are superior catalysts for the dehydrogenation of aliphatic hydrocarbons because few, if any, acidic sites are generated during the reduction of the catalysts, which reduction occurs either during the activation step prior to use, or during the dehydrogenation reaction. In contrast, a catalyst having a large amount of platinum will likely form a large number of acidic sites after platinum ions on the sieve are reduced to platinum metal. These acidic sites can cause coking and a reduction in the selectivity of the catalyst.

The catalysts described herein prepared from molecular sieves having a low platinum exchange capacity also have a high dispersion value of at least about 20%. Dispersion is a measure of the accessibility of the catalytic metals on the catalyst. Such dispersion methods are discussed in H. C. Gruber's, Analytical Chemistry, Vol. 13, p. 1828, 1962. The catalysts of this invention were analyzed for dispersion using a pulsed-carbon monoxide technique as described in more detail in the Examples. Platinum-containing catalysts having large dispersion values are desired because more of the platinum metal is available for reaction with the substrate molecules.

Platinum-loaded catalysts of this invention can contain 0.002 wt.% platinum to less than 0.01 wt.% platinum.

The crystalline molecular sieve useful in this invention can be admixed with or incorporated within various binders or matrix materials depending upon the intended process use. The crystalline sieve can be combined with active or inactive materials, synthetic or naturally-occurring zeolites, as well as inorganic or organic materials which would be useful for binding the sieve. Well-known materials include low acidity binders such as silica, alpha-alumina, magnesia, zirconia or other binders well-known in the art. Preferably, silica or calcined, low surface area alpha-alumina is used. A silica binder is most preferred. Typically, the sieve is incorporated within a matrix material by blending with a sol of the matrix material and gelling the resulting mixture. Also, solid particles of the sieve and matrix material can be physically admixed. Typically, such sieve compositions can be pelletized or extruded into useful shapes. The crystalline aluminosilicate content can vary anywhere from a few up to 100 wt.% of the total composition. Catalytic compositions can contain 0.1 wt.% to 100 wt.% crystalline sieve material and preferably contain 10 wt.% to 95 wt.% of such material and most preferably contain 20 wt.% to 80 wt.% of such material.

The preferred platinum-treated sieves of this invention exhibit an X-ray diffraction pattern comprising the following interplanar spacings and assigned strengths:

| Interplanar Spacing⁽¹⁾ d(Angstroms) | Assigned⁽²⁾ Strength | Interplanar Spacing⁽¹⁾ d(Angstroms) | Assigned⁽²⁾ Strength |
|---|---|---|---|
| 11.076 ± 0.05 | MS | 3.810 ± 0.04 | MW |
| 9.890 ± 0.05 | VS | 3.792 ± 0.04 | MW |
| 4.953 ± 0.04 | MW | 3.129 ± 0.04 | W |
| 3.841 ± 0.04 | MW | 1.985 ± 0.02 | W |

| | | | |
|---|---|---|---|
| (1) Copper K alpha radiation | | | |
| (2) VW = very weak, W = weak, M = medium, MS = medium strong, S = strong, VS = very strong | | | |

Prior to using the platinum-loaded sieve as a catalyst for the dehydrogenation of alkanes to olefins, the sieve is typically heat-treated or calcined. This heat treatment or calcination can occur in the reactor at reactor conditions used to conduct the dehydrogenation reaction. It can also be accomplished in a separate oven or furnace. For example, the platinum-treated sieve can be loaded into a reactor and heat-treated by being brought to reaction temperature in air or other oxygen-containing gas and then treated with hydrogen and/or the reaction feed mixture. In a typical heat-treatment procedure, the platinum-treated sieve is heated slowly in air to about 350°C to about 500°C and held there for about 1 to about 20 hrs. The platinum-treated sieve is then heated to about 500°C to about 600°C at about 40-60°C per hour and held at this second temperature in air for about 1 to about 10 hrs. The sieve is then cooled to about 350°C to about 450°C and reduced with hydrogen.

The platinum-loaded, molecular sieve supported in a low acidity matrix may be used as such or sulfided prior to or during use for catalysis by, for example, passing a gas stream containing dilute hydrogen sulfide over the catalyst surface while in the reactor.

Dehydrogenation in the presence of the above-described catalyst compositions is effected by contact of, for example, propane at a temperature between about 300°C and about 700°C and preferably between about 400°C and about 600°C. The reaction generally takes place at atmospheric pressure, but the pressure may be within the approximate range of about 1 bar to about 0.1 bar. (101 - 10 kPa) Use of a diluent such as nitrogen mixed with the alkane to be dehydrogenated is possible but later must be separated from the olefinic product. Dehydrogenation in the absence of a diluent gas or vapor is suitably accomplished utilizing a weight hourly space velocity of between about 0.1 to about 100 and preferably between about 0.5 and about 50.

The reaction product of the invention consists mainly of olefins. The alkane to olefin conversions within the appropriate range of space velocities are essentially the thermodynamic equilibrium values for the dehydrogenation temperatures employed. Typically, selectivity to olefin is greater than about 90 wt.% based on grams formed per 100 g of feed converted basis or greater than 95% on a moles formed per 100 moles converted basis.

The following Examples will serve to illustrate certain specific embodiments of the herein disclosed invention.

### EXAMPLES

### General

All percentages are wt.% unless otherwise stated. All elemental analyses were done using the ICP (Inductively Coupled Plasma) technique.

Platinum dispersion values were obtained by a pulsed CO chemisorption technique after hydrogen reduction. In this procedure, the catalyst samples were calcined at 500°C for 1 hr, purged with helium at 500°C, reduced in hydrogen at 500°C for 1 hr, purged with helium and cooled to room temperature. Using a catalyst sample size of about 1.3 grams, the activated catalyst was dosed with 0.045 cc pulses of 10% carbon monoxide (CO), balance nitrogen, and the carbon monoxide uptake was measured by a thermal conductivity cell. Platinum dispersion values were calculated assuming one carbon monoxide molecule per platinum atom. Platinum loadings are wt.% platinum metal.

The aluminosilicate molecular sieves were made generally according to example 10b on p. 19 of B. A. Jacobs and J. A. Martens' Studies in Surface Science and Catalysis, Vol. 33, "Synthesis of High Silica Aluminosilicate Zeolites" Elsevier Press (1987), incorporated herein by reference.

Catalytic runs were carried out by passing hydrocarbon feed through about a 10 cm length of catalyst contained in a ceramic tube plug flow reactor (520 mm by 18 mm i.d.) fitted with a sliding thermocouple to monitor the temperature profile. The products of the dehydrogenation reactor were passed on line through a GC fitted with a flame ionization detector for hydrocarbon analysis and a thermal conductivity detector for permanent gases (H₂, N₂) and C₁-C₄ hydrocarbons.

### Example 1

A 4.8 g amount of sodium hydroxide was dissolved in 120 ml of doubly distilled (DD) water and 33.3 g of silica (Aerosil-380) added with continuous mixing to form a homogeneous gel. A 7.44 g amount of tetrapropylammonium bromide dissolved in 114 ml of DD water was added to the gel followed by a solution made from 0.0385 g of NaAlO₂·H₂O in 30 ml of DD water while continuously mixing the gel. Sulfuric acid (95%) was added to the mix until the pH was 10 followed by 100 ml of DD water added with thorough mixing.

The result was added then to a 200 ml stirred autoclave and heated with stirring at 150°C for 72 hr. The autoclave was cooled and the crystalline product filtered, washed, and dried at 80°C. The resulting sodium zeolite product was then calcined in air at 550°C for 16 hrs, and calcined at 650°C for an additional 8 hrs.

A 20 g portion of the calcined sodium zeolite was suspended in a solution of 0.1712 g of tetraamineplatinum (II) chloride in 10 L of DD water, stirred for 24 hr at ambient temperature, and then filtered, washed and dried to form a platinum-loaded, sodium zeolite.

The platinum-loaded, sodium-form aluminosilicate molecular sieve contains 45% Si, 500 ppm Al, 0.0095% Pt and 840 ppm Na. The Si/Al ratio is 900. The surface area by BET using nitrogen is 374 m²/g with a micropore area of 70 m²/g and a micropore volume of 0.0365 cc/g. CO chemisorption shows a platinum dispersion of 24%. Powder x-ray diffraction studies show the sieve has the MFI crystal structure with a unit cell volume of 5346 cubic Angstroms. (5.346 x 10⁻²⁷ m³)

The platinum-loaded, sodium zeolite was pelletized by compressing the powder under 375 kg/cm² (36.7 MPa) pressure and crushing the result to a powder and thereafter sieving the powder to about a 0.5 to 1.0 mm particle size. The powder was mixed with 1 to 5 times its volume of alumina for use as a dehydrogenation catalyst. Prior to use of the dehydrogenation catalyst, the platinum compound was reduced by first passing air over the platinum-containing molecular sieve and gradually raising the temperature at 30°C/hr to 400°C. Hydrogen was then passed over the sieve at 400°C.

The complete powder diffraction spectrum (except for reflections with intensities less than one) for the platinum-loaded, sodium form aluminosilicate molecular sieve is set forth below:

| d-spacing | Intensity | d-spacing | Intensity |
|---|---|---|---|
| 9.8904 | 100 | 2.0065 | 2 |
| 11.0763 | 37 | 5.0087 | 2 |
| 3.8410 | 19 | 2.9455 | 2 |
| 3.8104 | 18 | 3.3436 | 2 |
| 3.7924 | 18 | 3.0291 | 2 |
| 4.9532 | 17 | 5.5117 | 1 |
| 3.1285 | 11 | 3.0475 | 1 |
| 1.9847 | 10 | 3.9938 | 1 |
| 3.7269 | 9 | 4.3409 | 1 |
| 3.3049 | 8 | 3.2892 | 1 |
| 3.7436 | 8 | 3.4218 | 1 |
| 3.7017 | 8 | 4.5969 | 1 |
| 5.9676 | 5 | 3.2427 | 1 |
| 1.9168 | 5 | 2.5077 | 1 |
| 6.3228 | 4 | 2.7777 | 1 |
| 5.6917 | 3 | 1.9973 | 1 |
| 5.9045 | 3 | 2.4679 | 1 |
| 2.4801 | 3 | 2.6051 | 1 |
| 2.9779 | 3 | 2.3846 | 1 |
| 1.6350 | 3 | 2.7265 | 1 |
| 3.6141 | 3 | 5.3135 | 1 |
| 5.5500 | 3 | 2.4093 | 1 |
| 5.6574 | 3 | 5.3533 | 1 |
| 3.6433 | 3 | 4.0632 | 1 |
| 4.2417 | 2 | 1.7447 | 1 |
| 6.6636 | 2 | 5.1145 | 1 |
| Copper K_{α} radiation | | | |

### Example 2

An unsupported sample of catalyst of Example 1 was tested for dehydrogenation with several different hydrocarbon feeds. The results are shown in Table 1 below.

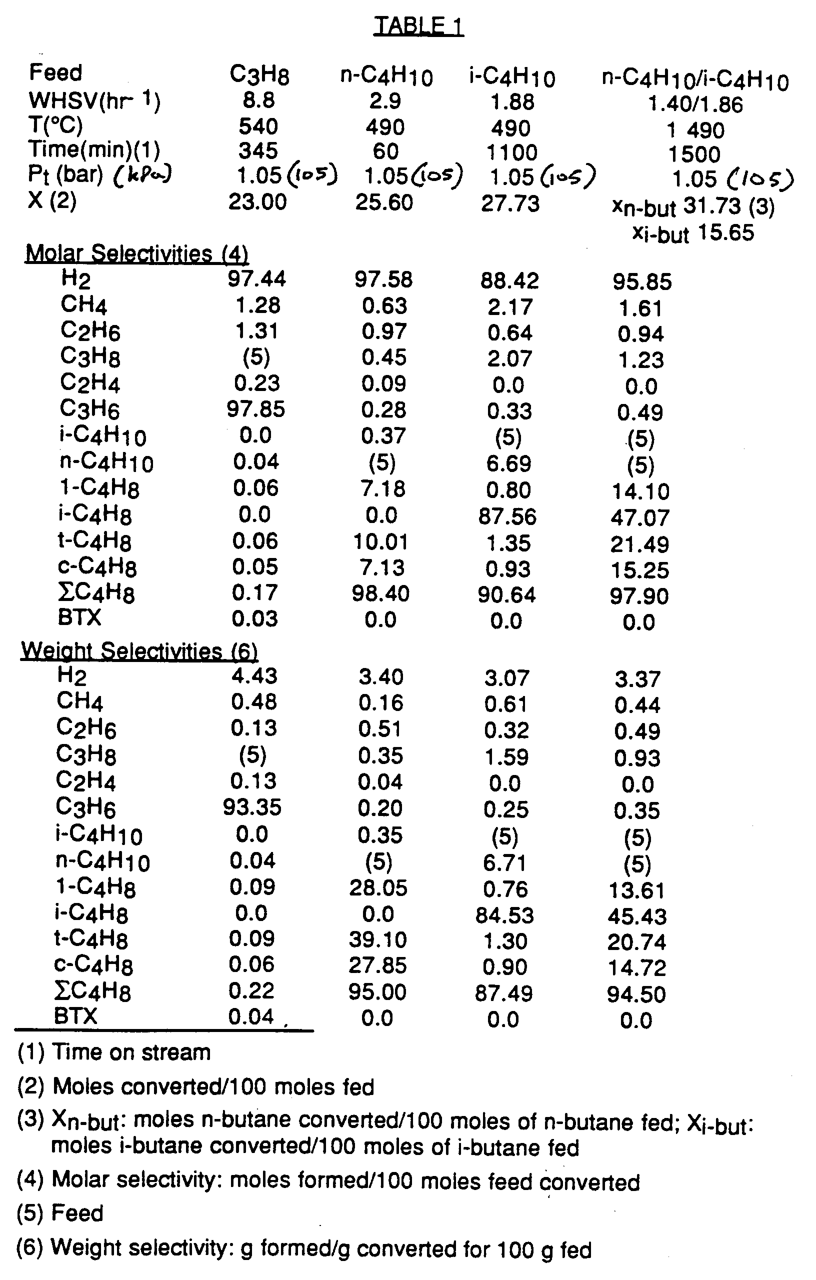

### Example 3

An unsupported sample of the catalyst of Example 1 was tested for dehydrogenation with both a C₃H₈ and a n-C₄H₁₀ feed, each feed diluted with nitrogen to reduce the hydrocarbon partial pressure to 0.1 bar (10 kPa). The catalytic results are shown below in Table 2.

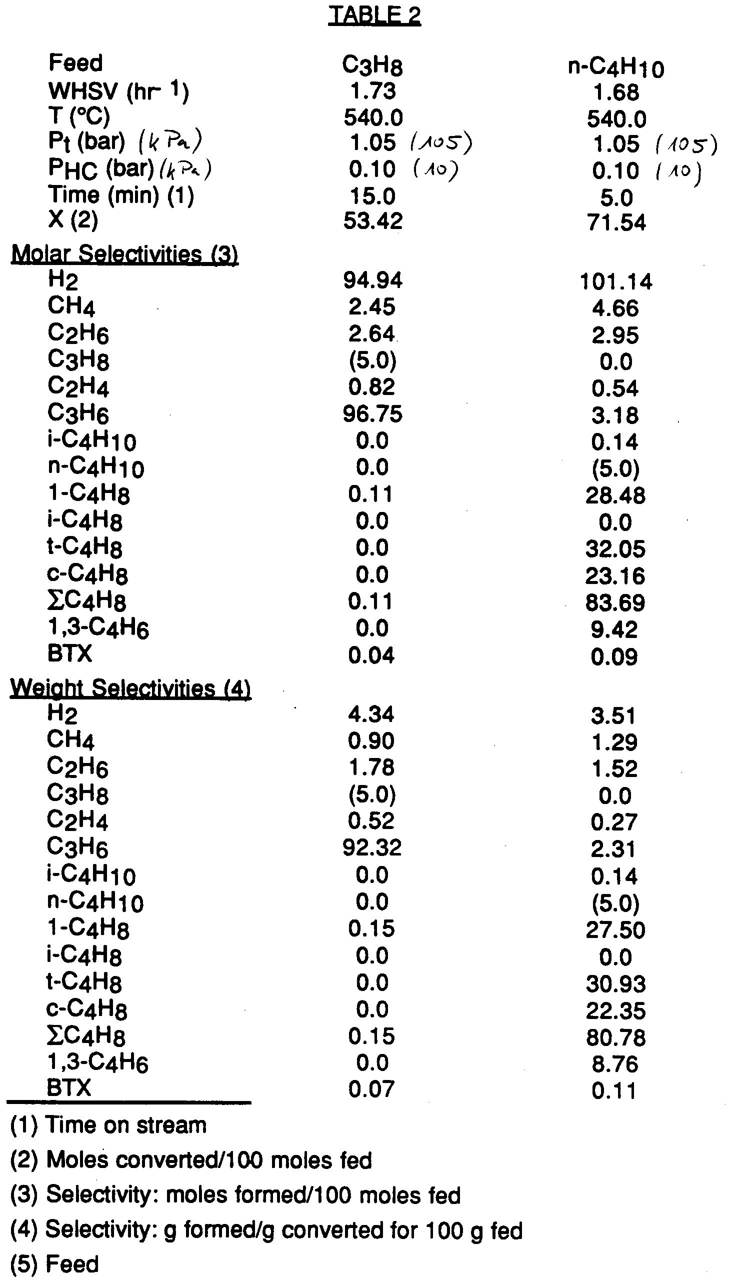

## Claims

1. A platinum-containing catalyst composition suitable for dehydrogenating C₃ to C₆ alkanes to alkenes, which composition contains, 0.002 wt%. to less than 0.01 wt% platinum and is prepared by the process comprising contacting a crystalline aluminosilicate sieve exhibiting the MFI crystal structure and having a platinum exchange capacity of no more than 500 parts per million by weight platinum as measured relative to the initial, platinum-unexchanged, sieve in its dry, calcined state by a procedure comprising contacting 20 grams of the sieve that has been previously calcined at 650°C for 8 hours, with a solution of 0.1712 grams of tetramineplatinum (II) chloride hydrate (0.005 gram platinum per gram of sieve) in 10 litres of doubly distilled water for 24 hours at room temperature, filtering the resulting sieve from the platinum-containing solution, washing with 1 litre of fresh doubly distilled water per gram of sieve, drying and measuring the amount of platinum in the resulting sieve, with at least one platinum compound to form a platinum-treated sieve; and thereafter heat-treating the platinum-treated sieve to form the platinum containing catalyst composition.

2. The catalyst composition of Claim 1 wherein the crystalline aluminosilicate sieve is in essentially the sodium form.

3. The catalyst composition of Claim 1 or Claim 2 where the crystalline aluminosilicate sieve has a silicon-to-aluminum atomic ratio of at least 800.

4. The catalyst composition of any one of Claims 1 to 3 wherein the crystalline aluminosilicate sieve has a platinum exchange capacity of no more than 200 parts per million by weight platinum.

5. The catalyst composition of any one of Claims 1 to 4 wherein the platinum-containing catalyst composition has a dispersion value of at least 20%.

6. The catalyst composition of any one of Claims 1 to 5 containing from 10 wt% to 95 wt. % of the crystalline aluminosilicate.

7. The catalyst composition of any one of Claims 1 to 6 wherein the crystalline aluminosilicate sieve has a silicon/aluminum ratio between 800 and 2000.

8. The catalyst composition of any one of Claims 1 to 6 wherein the crystalline aluminosilicate sieve has a silicon/aluminum ratio between 800 and 1500.

9. The catalyst composition of any one of Claims 1 to 8 wherein the crystalline aluminosilicate sieve is prepared in a crystallization vessel that does not introduce metal ions into the sieve.

10. A dehydrogenation process which contacts under hydrocarbon conversion conditions a feed comprising at least one C₃ to C₆ alkane with the platinum-containing catalyst composition of any one of Claims 1 to 9.

## Patentansprüche

1. Eine platinhaltige Katalysatorzusammensetzung, welche für die Dehydrierung von C₃- bis C₆-Alkanen zu Alkenen geeignet ist, wobei die Zusammensetzung 0,002 Gew.-% bis zu weniger als 0,01 Gew.-% Platin enthält und wobei diese hergestellt wird durch ein Verfahren, welches umfaßt: ein In-Kontakt-Bringen eines kristallinen Aluminosilicatmolekularsiebes, welches eine MFI-Kristallstruktur aufweist und eine Platinaustauschkapazität von nicht mehr als 500 Teilen pro Million pro Gewicht an Platin aufweist, gemessen in Bezug auf das anfängliche, nicht mit Platin ausgetauschte Molekularsieb in seinem trockenen, ausgeglühten Zustand durch ein Verfahren, das ein In-Kontakt-Bringen von 20 Gramm des Molekularsiebes, welches vorher 8 Stunden lang bei 650°C ausgeglüht wurde, mit einer Lösung von 0,1712 Gramm Tetramin-Platin(II)-chloridhydrat (0,005 Gramm Platin pro Gramm des Molekularsiebes) in 10 Litern doppelt destilliertem Wasser für 24 Stunden bei Raumtemperatur, ein Filtrieren des resultierenden Molekularsiebes aus der platinhaltigen Lösung, ein Waschen mit 1 Liter von frisch doppelt destilliertem Wasser pro Gramm des Molekularsiebes, ein Trocknen und Messen der Menge an Platin in dem resultierenden Molekularsieb umfaßt, mit wenigstens einer Platinverbindung, um ein mit Platin behandeltes Molekularsieb zu bilden; und danach ein Wärmebehandeln des mit Platin behandelten Molekularsiebes, um die platinhaltige Katalysatorzusammensetzung zu bilden.

2. Die Katalysatorzusammensetzung nach Anspruch 1, wobei das kristalline Aluminosilicatmolekularsieb im wesentlichen in der Natriumform vorliegt.

3. Die Katalysatorzusammensetzung nach Anspruch 1 oder Anspruch 2, wobei das kristalline Aluminosilicatmolekularsieb ein Atomverhältnis von Silicium zu Aluminium von wenigstens 800 aufweist.

4. Die Katalysatorzusammensetzung nach einem der Ansprüche 1 bis 3, wobei das kristalline Aluminosilicatmolekularsieb eine Platinaustauschkapazität von nicht mehr als 200 Teilen pro Million pro Gewicht an Platin aufweist.

5. Die Katalysatorzusammensetzung nach einem der Ansprüche 1 bis 4, wobei die platinhaltige Katalysatorzusammensetzung einen Dispersionswert von wenigstens 20% aufweist.

6. Die Katalysatorzusammensetzung nach einem der Ansprüche 1 bis 5, welche von 10 Gew.-% bis 95 Gew.-% des kristallinen Aluminosilicats aufweist.

7. Die Katalysatorzusammensetzung nach einem der Ansprüche 1 bis 6, wobei das kristalline Aluminosilicatmolekularsieb ein Verhältnis von Silicium/Aluminium zwischen 800 und 2000 aufweist.

8. Die Katalysatorzusammensetzung nach einem der Ansprüche 1 bis 6, wobei das kristalline Aluminosilicatmolekularsieb ein Verhältnis von Silicium/Aluminium zwischen 800 und 1500 aufweist.

9. Die Katalysatorzusammensetzung nach einem der Ansprüche 1 bis 8, wobei das kristalline Aluminosilicatmolekularsieb in einem Kristallisationsgefäß hergestellt wird, das keine Metallionen in das Molekularsieb einführt.

10. Ein Dehydrierungsverfahren, welches unter Kohlenwasserstoffumwandlungsbedingungen ein Beschickungsmaterial, das wenigstens ein C₃- bis C₆-Alkan umfaßt, mit der platinhaltigen Katalysatorzusammensetzung aus einem der Ansprüche 1 bis 9 in Kontakt bringt.

## Revendications

1. Composition catalytique contenant du platine, appropriée à la déshydrogénation d'alcanes en C₃ à C₆ en alcènes, laquelle composition contient de 0,002 % en poids à moins de 0,01 % en poids de platine et est préparée par le procédé comprenant la mise en contact d'un tamis d'aluminosilicate cristallin présentant la structure de cristal MFI et ayant une capacité d'échange n'excédant pas 500 parties par million en poids de platine mesurée par rapport au tamis initial sans platine échangé, dans son état sec et calciné, selon une procédure comprenant la mise en contact de 20 grammes de tamis préalablement calciné à 650°C pendant 8 heures, avec une solution de 0,1712 grammes de chlorure de platine(II)tétramine hydraté (0,005 gramme de platine par gramme de tamis) dans 10 litres d'eau bidistillée pendant 24 heures à température ambiante, la filtration du tamis ainsi obtenu à partir de la solution contenant du platine, le lavage avec 1 litre d'eau fraîchement bidistillée par gramme de tamis, le séchage et la mesure de la quantité de platine dans le tamis ainsi obtenu, avec au moins un composé du platine pour former un tamis traité au platine ; et ensuite le traitement par la chaleur du tamis traité au platine pour former la composition catalytique contenant le platine.

2. Composition catalytique selon la revendication 1, dans laquelle le tamis d'aluminosilicate cristallin est sous la forme essentiellement sodique.

3. Composition catalytique selon la revendication 1 ou la revendication 2, dans laquelle le tamis d'aluminosilicate cristallin a un rapport atomique silicium/aluminium d'au moins 800.

4. Composition catalytique selon la revendication 1 ou la revendication 2, dans laquelle le tamis d'aluminosilicate cristallin a une capacité d'échange de platine n'excédant pas 200 parties par million en poids de platine.

5. Composition catalytique selon l'une quelconque des revendications 1 à 4, dans lequel la composition catalytique contenant du platine a une valeur de dispersion d'au moins 20 %.

6. Composition catalytique selon l'une quelconque des revendications 1 à 5, contenant de 10 % en poids à 95 % en poids d'aluminosilicate cristallin.

7. Composition catalytique selon l'une quelconque des revendications 1 à 6, dans lequel le tamis d'aluminosilicate cristallin a un rapport silicium/aluminium compris entre 800 et 2.000.

8. Composition catalytique selon l'une quelconque des revendications 1 à 6, dans lequel le tamis d'aluminosilicate cristallin a un rapport silicium/aluminium compris entre 800 et 1.500.

9. Composition catalytique selon l'une quelconque des revendications 1 à 8, dans lequel le tamis d'aluminosilicate cristallin est préparé dans une cuve de cristallisation qui n'introduit pas d'ions métalliques dans le tamis.

10. Procédé de déshydrogénation qui met en contact dans des conditions de conversion d'hydrocarbures, un produit de départ comprenant au moins un alcane en C₃ à C₆ avec la composition catalytique contenant du platine selon l'une quelconque des revendications 1 à 9.
